# EUROPEAN PATENT APPLICATION

(11) **EP 3 546 570 A2**
(43) Date of publication of application: **02.10.2019**
(21) Application number: 17872943.0
(22) Date of filing: 16.11.2017
(51) Int. Cl.: C12N 5/078, C12N 5/00, A61K 8/98, A61K 35/17, A61Q 19/00

(54) **SERUM-FREE IMMUNE CELL-CULTURING MEDIUM-ADDED KIT, METHOD FOR CULTURING IMMUNE CELLS BY USING SAME KIT, SERUM-FREE IMMUNE CELL CULTURE OBTAINED BY SAME KIT OR CULTURING METHOD, AND COSMETIC COMPOSITION COMPRISING SAME CULTURE**

(30) Priority: 22.11.2016 KR 20160155890
(71) Applicant: Nkbio Tech Co., Ltd., Jungwon-gu, Seongnam-si Gyeonggi-do 13201 (KR)
(72) Inventor: SHIN, Dong Hyuk, Gyeonggi-do 13527 (KR); KWAK, Dong Seob, Suwon-si Gyeonggi-do 16435 (KR)
(74) Representative: Vossius & Partner Patentanwälte Rechtsanwälte mbB
(86) International application number: PCT/KR2017/013020
(87) International publication number: WO 2018/097540

(57) **Abstract**

The present invention relates to a technology for culturing Natural Killer cells (NK cells) applied to immunotherapy, and more particularly, a kit for adding to the serum-free immune cell culturing media capable of effectively amplifying and activating immune cells that have been left for one day or longer after blood sampling or have become much weakened, while culturing NK cells to remarkably increase the portion of NK cells therein, compared with conventional immune cell-culturing methods, a method for culturing immune cells by using the kit, a serum-free immune cell cultured medium obtained using the kit or the culturing method, and a cosmetic composition comprising the cultured medium.

## Description

### TECHNICAL FIELD

The present invention relates to a technology for culturing Natural Killer cells (NK cells) applied to immunotherapy, and more particularly, a kit for adding to the serum-free immune cell culturing media that allows to determine the use of serum according to the purpose after effectively amplifying and activating immune cells left for one day or longer after blood sampling or much weakened, while remarkably increasing the portion of NK cells, compared with conventional immune cell-culturing methods, and optionally to culture in the presence or absence of serum, a culturing method for immune cells by using the kit, a serum-free immune cell cultured medium obtained by the kit or the culturing method, and a cosmetic composition comprising the cultured medium.

### BACKGROUND ART

The mammalian immune system is composed of a number of unique cells that function to defend the host from infected bacteria, viruses, toxins, and other non-host substances. The cell type that plays a major role in the specificity of the immune system is called lymphocytes, which include B cells, T cells and NK cells, that is, natural killer cells. T cells are so named because they are produced in thymocyte, B cells are so named because they are produced in the bone marrow, and NK cells are named as Natural Killer (NK) cells because they are born with the ability to precisely distinguish themselves from others from the time that they are born. T-cell population includes several subtypes, such as suppressor T cells, cytotoxic T cells, and T helper cells. The T-helper cell population determines two immune pathways, Th1 and Th2. Th1 cells (a functional subpopulation of CD4 + cells) are characterized by their ability to increase the cell-mediated immune response. Th1 cells produce cytokine IL-2 and interferon-γ. Th2 cells are also CD4 + cells, but different from Th1 cells. Th2 cells are responsible for antibody production and produce cytokines IL-4, IL-5, IL-10 and IL-13. These cytokines play an important role for Th1 and Th2 responses to interfere each other.

Among the immune cells that are activated by immunotherapy, especially, NK cells that are large granular lymphocytes (LGLs), a characteristic form of lymphocytes, have an excellent ability to kill infected viruses and tumor cells, and characteristically, do not kill most normal cells. The antitumor activity is obtained by necrosis, apoptosis, or both mechanisms of action occurring concurrently. NK cells respond to cytokines, such as IL-2, IL-12, interferon and the like, which enhance cytotoxicity, secretory, and proliferative functions. The phenotype of NK cells is CD16 (FcγRIII) and CD56 in humans, and CD16 and CD56 are used as markers for NK cells, because the cell surface does not contain TRC (T-cell receptor complex).

Such NK cells are known to play an important role in early biological defense mechanisms and tumor immunity of the human body. In other words, NK cells can kill specific auto-cells, allogeneic cells, and even xenogeneic cancer cells without the acquired immunity process by the expression of major histocompatibility complex (MHC), and especially, can kill target cells that express less or do not express Class 1 MHC. Thus, NK cells can effectively kill most cancer cells that do not express MHC as well as some virus-infected cells and bacteria, such as salmonella typhi.

However, NK cells, which exert an excellent effect of killing cancer cells, as described above, account for only 5-25% of peripheral blood lymphocytes even in the case of normal human. In particular, in the case of cancer patients, the proportion is reduced to less than 5%, so there is a limit to effectively attack cancer cells without a separate amplification process through immunotherapy.

For activation and proliferation of immune cells, particularly NK cells, cytokines, such as IL-2 and the like, and antibodies, such as CD3 and the like, are used. In current technologies, the CD3 antibody plays a very important role in the proliferation of immune cells, yet the problem is that it is very difficult to activate immune cells using this antibody. That is, the current common technique for culturing immune cells is to immobilize the CD3 antibody in a flask and stimulate cells for a certain period of time. However, for immune cells, each individual has distinctive susceptibility and it can be greatly varied depending on cell culture conditions or the skill level of the operator. For example, when the stimulation of the CD3 antibody is done too less or is almost not done, the immune cells do not proliferate well. In addition, when the stimulation is done too much, NK cells hardly proliferate and NKT cells or T cells proliferate too much. In this case, since T cells are proliferated to much, it is not easy to proliferate and obtain a large number of NK cells.

In particular, if strong stimulation of the CD3 antibody is applied from the beginning, immature progenitor cells will mature into T cells. Therefore, the current method generally used employs the procedure of slightly stimulating the CD3 antibody at the beginning. However, it is difficult to obtain activated NK cells that are proliferated in a large amount, because it is highly influenced by environmental conditions, such as individual differences and the like.

Meanwhile, when immune cells are cultured *in vitro* using a high concentration of IL-2 and antibodies, such as CD3, CD16, CD56 and the like, after separating lymphocytes from the peripheral blood, various cytokines are secreted while NK cells and Th1 type cells are proliferated, and immune cells are divided and grow. These cytokines (γ-interferon, IL-2, IL-8, IL-12, IL-18, *etc*.) inhibit the overproduction of Th2 cytokines as well as improve skin immunity, and thus can be used very effectively for the treatment of skin inflammation.

Among these cytokines, IL-8, which is produced in large amounts in NK cells, induces aggregation of mononuclear cells in the blood and differentiation of them into macrophages. The macrophages first act to remove antigens, but when no more antigens to be removed are present, or by the action of IL-4, the macrophages are converted to the M2a type. The M2a-type macrophages produce several kinds of related factors (TGF-β, bFGF, PDGF, VEGF, MMPs, TIMPs) to restore tissues destroyed by wound or inflammation. Ultimately, by increasing macrophages (M2a type) by IL-8, which is produced in large amounts in NK cells, treatment of inflammation or wound can be quickly treated. Furthermore, IL-8 can be effective in improving or treating skin disease by maintaining the epidermis strong and healthy through collecting keratinocytes to the epidermis, and thus effectively preventing external infiltration. In addition, when IL-8 alone or IL-8 and IL-4 or interferon γ and the like obtained through easily available genetic recombination technology are used together, macrophages significantly proliferated by IL-8 are converted to M2a-type macrophages by IL-8, and thus the wound-treating effect and the skin care effect can be improved.

However, when immune cells are cultured according to the known culturing method for immune cells, the rate of immune cell death becomes faster than the rate of immune cell proliferation after 2 weeks from the starting point of culture, and after 3 to 4 weeks, immune cells are hard to find in the culture media. Therefore, immune cells are usually obtained by extracting lymphocytes from the blood, culturing the extracted lymphocytes for 2 weeks to obtain proliferated immune cells, and using the obtained immune cells or the cultured media as it is or after freezing for storage. Consequently, to obtain a large number of immune cells or a large amount of immune cell cultured media, there is a problem of requiring a large amount of blood.

In addition, serum is generally used in immune cell culture. In this case, when the cultured media obtained by using serum of another person or animal are used as a cosmetic composition or therapeutic agent, the problem of the reluctant use exists because of the risk of infection of any unknown diseases due to the use of serum. Hence, the culture media without serum is desperately required.

Moreover, the validity of immune cells to be used is within 24 hours after collecting the blood. That is, the lifespan is short and after one day post-blood collection, the cells are rapidly weakened, and cell culture becomes difficult. These problems make it difficult to obtain the donated blood. To recover and culture immune cells left for long time after blood sampling or obtained from a person with the reduced immunity, or immune cells left in a leukocyte removal filter that are discarded in the preparation of the transfusion blood, a method capable of activating and culturing weakened immune cells is required.

### DETAILED DESCRIPTION OF THE INVENTION

### Technical Problem

As the consequence of efforts to solve the above problems, the inventors of the present invention completed the present invention by developing a method for stably amplifying NK cells in lymphocyte culture media without performing the step of incubating for a certain period of time after fixing the anti-CD3 antibody, followed by removing the antibody, and furthermore, from originally weak immune cells or, for example, the immune cell from the elderly or even the immune cells of cancer patients.

Accordingly, one purpose of the present invention is to provide a kit for adding to the serum-free immune cell culturing media containing a composition that allows to determine whether serum would be optionally included in the mass-culture process to culture, without the inconvenient steps of immobilizing the anti-CD3 antibody in a culture container and culturing the lymphocytes (immune cells) for a certain period of time to stimulate lymphocytes, and at the same time, is capable of stably amplifying and proliferating NK cells in a large amount in the finally obtained lymphocyte cultured media.

Another purpose of the present invention is to provide a method for stably culturing immune cells in which the finally obtained lymphocyte cultured media contain the low proportion of T cells and the remarkably high proportion of NK cells even when originally weak immune cells or immune cells of, for example, the elderly or severe cancer patients are cultured, by sequentially using the additives constituting units contained in the kit for adding to the media, *i.e*., by specifying the sequence of stimulating lymphocytes.

Another purpose of the present invention is to provide a kit for adding to the serum-free immune cell culturing media capable of culturing NK cells very easily by standardizing the medium additives to be added during the culturing process in order to proliferate NK cells at a remarkably high proportion in the lymphocyte culture.

Another purpose of the present invention is to provide a cosmetic composition highly effective for whitening the skin or improving the skin wrinkles via the skin regeneration effect containing the serum-free immune cell cultured media with high concentrations of interleukin-8, interferon γ, and the like as an effective ingredient.

Still another purpose of the present invention is to provide a pharmaceutical composition highly effective for wound healing, such as injuries, burn and the like, and the treatment of skin diseases containing the serum-free immune cell cultured media with high concentrations of interleukin-8, interferon γ, and the like as an effective ingredient.

The purpose of the present invention is not limited to the above-described purposes, and although not explicitly mentioned, the purpose of the present invention that can be recognized by a person ordinarily skilled in the art from the detailed description of the present invention as described below can be obviously included.

### Technical Solution

To achieve the above-described purpose of the present invention, the present invention provides a kit for adding to the serum-free immune cell culturing media containing B unit composed of the basic solution including IL-2, L-glutamine and cell culture media; C1-1 unit composed of the cytokine 1-1 solution formed by dissolving IL-12 and IL-18 in the basic solution, such that IL-12 is included at a concentration of 0.5-5 ng/mL and IL-18 is included at a concentration of 2-50 ng/mL; C1-2 unit composed of the cytokine 1-2 solution formed by dissolving IL-12 and IL-18 in the basic solution, such that IL-12 is included at a concentration of 5.1-15 ng/mL and IL-18 is included at a concentration of 30-120 ng/mL; C2 unit composed of the cytokine 2 solution formed by dissolving IL-15 in the basic solution, such that IL-15 is included at a concentration of 10-50 ng/mL; A1 unit composed of the antibody 1 solution formed by dissolving the anti-CD16 and the anti-CD56 in the basic solution, such that the anti-CD16 and the anti-CD56 are included at a concentration of 0.1-15 µg/mL, respectively; A2 unit composed of the antibody 2 solution containing the antibody 1 solution:the basic solution in a volume ratio of 1:6-10; and D unit composed of the antibody-cytokine mixture solution formed by dissolving the anti-CD3 in the cytokine 1 solution, such that the anti-CD3 is included at a concentration of 1-12 µg/mL.

In a preferred embodiment, A1 unit is added to the lymphocyte culture media prior to D unit.

In a preferred embodiment, the anti-CD16, the anti-CD56, and the basic solution contained in A1 unit are separately packaged and mixed in the step of adding to the media to form the antibody 1 solution.

In a preferred embodiment, the anti-CD16, the anti-CD56 and the basic solution of the antibody 1 solution contained in A2 unit and the basic solution are separately packaged and mixed in the step of adding to the media to form the antibody 2 solution.

In a preferred embodiment, the anti-CD3 and the cytokine 1 solution contained in D unit are separately packaged and mixed in the step of adding to the media to form the antibody-cytokine mixture solution.

In a preferred embodiment, the units are composed to be sequentially added to the lymphocyte culture media and used in order of C1-1 unit, C2 unit, A1 unit, D unit, C1-1 unit, A2 unit, C2 unit, A2 unit, B unit and C1-2 unit, order of C2 unit, C1-1 unit, A1 unit, D unit, C1-1 unit, A2 unit, C2 unit, A2 unit, B unit and C1-2 unit, order of C1-1 unit, A1 unit, D unit, C1-1 unit, A2 unit, C2 unit, A2 unit, B unit and C1-2 unit, or order of C2 unit, A1 unit, D unit, C1-1 unit, A2 unit, C2 unit, A2 unit, B unit and C1-2 unit.

The present invention also provides a method for culturing immune cells including the first step of adding at least one of the cytokine 1-1 solution of C1-1 unit and the cytokine 2 solution of C2 unit to the isolated lymphocytes, followed by addition of the autologous plasma; the second step of adding the antibody 1 solution of A1 unit to the media to which the first step is performed; the third step of adding the antibody-cytokine mixture solution of D-unit to the media to which the second step is performed; the fourth step of adding the cytokine 1-1 solution of C1-1 unit to the media to which the third step is performed, followed by addition of the autologous plasma; the fifth step of adding the antibody 2 solution of A2 unit to the media to which the fourth step is performed, followed by addition of the autologous plasma or FBS (fetal bovine serum); the sixth step of adding the cytokine 2 solution of C2 unit to the media to which the fifth step is performed, followed by addition of the autologous plasma or FBS; and the seventh step of adding the antibody 2 solution of A2 unit and the autologous plasma or FBS to the media to which the sixth step is performed.

In a preferred embodiment, the method further includes the eighth step of adding the basic solution of B unit and the autologous plasma or FBS to the media to which the seventh step is performed, and culturing; and the ninth step of adding the autologous plasma or FBS to the media to which the eighth step is performed, placing into a large-capacity cell culture container containing cell culture media, and culturing.

In a preferred embodiment, the isolated lymphocytes are isolated from the blood left for one day or more after blood collection.

In a preferred embodiment, the method further includes the tenth step of separating the media to which the seventh step or the ninth step is performed to cells and serum-containing cultured media; the eleventh step of washing the cells separated in the tenth step; the twelfth step of placing the washed cells in the eleventh step into a new culture container, adding the basic solution of B unit, and culturing; and the thirteenth step of placing the media to which the twelfth step is performed into a large-capacity cell culture container containing cell culture media, adding the cytokine 1-2 solution of C1-2 unit, and culturing.

In a preferred embodiment, the method further includes the fourteenth step of separating the media to which the thirteenth step is performed to cells and primary serum-free cultured media; and the fifteenth step of adding the basic solution of B unit to the cells separated in the fourteenth step, placing the cells into a large-capacity cell culture container containing cell culture media, adding the cytokine 1-2 solution of C1-2 unit, and culturing.

In a preferred embodiment, the autologous plasma contains more than 40 usp units of heparin per 1 ml of plasma.

In a preferred embodiment, the content of the autologous plasma or FBS added is less than 10% by volume of the total media.

The present invention also provides the immune cell cultured media cultured by any one of the above-described culturing methods.

The present invention also provides the serum-free immune cell cultured media obtained from the immune cell cultured media cultured using any of the above-described kits for adding to the immune cell culturing media or by any one of the above-described culturing methods.

The present invention also provides a cosmetic composition containing the above-described serum-free immune cell cultured media; and a cosmetic base.

In a preferred embodiment, the serum-free immune cell cultured media function as an active ingredient for at least one of inflammation improvement, skin whitening, pigmentation removal and skin regeneration.

In a preferred embodiment, the serum-free immune cell cultured media is contained in an amount of 50% by weight or less based on the weight of the total composition.

The present invention also provides a pharmaceutical composition for the treatment of wounds including burn and injuries or skin diseases containing the above-described serum-free immune cell cultured media.

### Effects of the Invention

The present invention has the following excellent effects.

First of all, the kit for adding to the immune cell culturing media of the present invention eliminates the cumbersome process of culturing lymphocytes (immune cells) for a certain period of time to stimulate lymphocytes, while immobilizing the anti-CD3 antibody in a culture container, and in the case of large scale of culture, it can be selectively determined whether culture is performed in the presence or absence of serum, and at the same time, NK cells can be stably amplified and proliferated in a large amount in the finally obtained lymphocyte cultured media.

In addition, the immune cell culturing method of the present invention can decrease the proportion of T cells and remarkably increase the proportion of NK cells in the finally obtained lymphocyte cultured media, even when originally weak immune cells or immune cells of, for example, the elderly or severe cancer patients are cultured, by sequentially using the additives constituting units contained in the kit for adding to the media, *i.e*., by specifying the sequence of stimulating lymphocytes.

In addition, according to the present invention, NK cells can be cultured very easily by standardizing the medium additives to be added during the culturing process in order to proliferate NK cells at a remarkably high proportion in the lymphocyte culture.

Further, according to the cosmetic composition of the present invention, the effect of skin whitening, improvement of the skin wrinkles via the skin regeneration effect, and the like is excellent, as the serum-free immune cell cultured media with high concentrations of interleukin-8, interferon γ, and the like are contained as an effective ingredient.

Further, according to the pharmaceutical composition of the present invention, the effect of wound healing, such as injuries, burn and the like, the treatment of skin diseases, and the like is excellent, as the serum-free immune cell cultured media with high concentrations of interleukin-8, interferon γ, and the like are contained as an effective ingredient.

The technical advantages of the present invention are not limited to the above-described scopes, and although not explicitly mentioned, the effect of the present invention that can be recognized by a person ordinarily skilled in the art from the detailed description for the examples of the present invention as described below can be obviously included.

### BRIEF DESCRIPTION OF DRAWINGS

FIGS. 1 and 2 are progressive photographs showing the effects of improving the inflammation and removing the pigmentation obtained by applying the cosmetic composition according to an embodiment of the present invention to the area with acne.
FIG. 3 is progressive photographs showing the effects of improving the inflammation and removing the pigmentation obtained by applying the cosmetic composition according to an embodiment of the present invention to a red spot formed from bitten by an insect alive.
FIG. 4 is progressive photographs showing the effects of improving the inflammation and removing the pigmentation obtained by applying the cosmetic composition according to an embodiment of the present invention to the area with an incidence of allergy.
FIG. 5 is progressive photographs showing the effects of improving the inflammation and removing the pigmentation obtained by applying the cosmetic composition according to an embodiment of the present invention to the wide area where several allergies occur at different time intervals.
FIG. 6 is progressive photographs showing the skin regeneration effect obtained by applying the pharmaceutical composition according to an embodiment of the present invention to a burn area.

### MODE OF EMBODIMENTS OF THE INVENTION

The terms used in the present invention is solely for the purpose of describing particular embodiments and is not intended to limit the invention. The singular expression includes the plural expression unless the context clearly indicates otherwise. In the present application, the terms "comprising" or "having" and the like are intended to indicate the presence of features, numbers, steps, operations, elements, parts, or combinations thereof as described in the application, but not intended to preclude the presence or addition of one or more other features, numbers, steps, operations, elements, parts, or combinations thereof.

The terms first, second, *etc*. may be used to describe various elements, but the elements should not be limited by the terms. The terms are used solely for the purpose of distinguishing one component from another component. For example, without leaving the scope of the right of the present invention, the first component may be referred to as a second component, and similarly, the second component may also be referred to as a first component.

Unless defined otherwise, all terms used herein, including technical or scientific terms, have the same meaning as commonly understood by one of ordinary skill in the art to which this invention pertains. The terms such as those defined in commonly used dictionaries are to be interpreted as having a meaning consistent with the meaning of the context in the relevant art and, unless expressly defined in the present invention, are not to be interpreted with ideal or overly formal meaning.

As used in the present invention, the term "serum-free immune cell cultured media" means the residue obtained by separating immune cells from the immune cell culture media in the absence of serum. Therefore, the serum-free immune cell cultured media can be obtained by separating the immune cells from the immune cell culture media amplified in the absence of serum, and include secondary metabolites of immune cells, such as cytokines. In addition, "NK cells" was used to include both NK cells and NKT cells.

Hereinafter, the technical structure of the present invention will be described in detail with reference to the accompanying drawings and preferred embodiments.

However, the present invention is not limited to the embodiments described herein but may be embodied in other modes. The same reference numbers used to describe the present invention throughout the specification identify the same elements.

The technical feature of the present invention is a kit for adding to the serum-free immune cell culturing media containing a composition that allows to determine whether serum would be optionally included in the mass-culture process to culture, without the cumbersome steps of immobilizing the anti-CD3 antibody in a culture container and culturing the lymphocytes (immune cells) for a certain period of time to stimulate lymphocytes, and is capable of stably amplifying and proliferating NK cells in a large amount in the finally obtained lymphocyte cultured media, even when originally weak immune cells or immune cells of, for example, the elderly or severe cancer patients are cultured, a culturing method of using the kit for adding to the serum-free immune cell culturing media, and a method of using the product obtained from the cultured media.

In general, when the NK cell-based immune cells are cultured, antibodies, such as CD3, CD16 and CD56, are conventionally used. If the cells weakened are directly stimulated by the antibodies, the cells often die more quickly. It appears that apoptosis is induced when the cells weakened are strongly stimulated directly.

To solve this problem, the present invention is focused on the fact that, when the NK cell-based immune cells are cultured, the cells proliferate well without dying if the weakened cells are first strongly activated and then, stimulated by antibodies.

In other words, it is identified that when NK cell-based immune cells are cultured, many cytokines, including interferon γ and the like, are secreted and immune cells are greatly recovered and activated by the secreted cytokines if cells are first stimulated with cytokines, such as a mixture of IL-12 and IL-18, a mixture of IL-12 and IL-15, or IL-15 alone, in the presence of a certain concentration of IL-2, for 12-24 hours or longer before stimulation with antibodies. It is also identified that NK cells, NKT cells, T cells and the like proliferate well, as intended, when the cells are stimulated with antibodies (stimulated with the anti-CD16 and the anti-CD56, followed by the anti-CD3 stimulation or stimulated only with the anti-CD3) in the activated state. At this time, the immune cells can be stimulated simply by adding antibodies to the media without using the immobilized antibodies.

In accordance with the principles developed in the present invention, immature progenitor cells that have not yet matured are stimulated with the anti-CD16 and the anti-CD56 antibodies to induce differentiation into NK cells. If the T cells activated by stimulating T cells with the anti-CD3 antibody activate NK cells after activation of the already matured NK cells, NK cells greatly proliferate without any problems by continuous stimulation until the anti-CD3 antibody is degraded and disappears in the culture media. Based on the experimental results, in the culturing method of the present invention, the lymphocytes can be cultured with a very high proportion of NK cells without the cumbersome process of culturing lymphocytes (immune cells) for a certain period of time, while the anti-CD3 antibody is immobilized in the culture container to stimulate lymphocytes.

Using this method, immune cells can be cultured well, even if they are originally weak immune cells or immune cells of, for example, the elderly or severe cancer patients. In particular, while leukocytes are filtered and removed in the preparation of the transfusion blood, it is very useful for recovering and culturing lymphocytes in the leukocyte removal filter to be discarded.

Therefore, the kit for adding to the serum-free immune cell culturing media of the present invention is individually packaged so that it can be added to the media at each step of the lymphocyte culture, and contains B unit, C1-1 unit, C1-2 unit, C2 unit, A1 unit, A2 unit and D unit with distinguishable contents, *i.e*., distinguishable elements. Here, elements to be included in the units are determined in consideration of the influence on the lymphocyte culture media, such that most of the cultured cells are NK cells, even when the weakened lymphocytes are cultured in the lymphocyte culture. In particular, the kit for adding to the serum-free immune cell culturing media of the present invention is configured to follow any one of the following four orders of adding the units to the lymphocyte culture media to use, such that NK cells can be stably proliferated at least 500 to 5000 times as compared with the conventional culture methods when lymphocytes isolated from the blood left for one day or longer after blood collection are cultured.
(1) order of C1-1 unit, C2 unit, A1 unit, D unit, C1-1 unit, A2 unit, C2 unit, A2 unit, B unit and C1-2 unit (2) order of C2 unit, C1-1 unit, A1 unit, D unit, C1-1 unit, A2 unit, C2 unit, A2 unit, B unit and C1-2 unit; (3) order of C1-1 unit, A1 unit, D unit, C1-1 unit, A2 unit, C2 unit, A2 unit, B unit and C1-2 unit, (4) order of C2 unit, A1 unit, D unit, C1-1 unit, A2 unit, C2 unit, A2 unit, B unit and C1-2 unit.

First, B unit is composed of a basic solution, which contains IL-2, L-glutamine and cell culture media. In this case, the content of IL-2 may be 100 ng/mL-300 ng/mL, preferably at the concentration of 200-250 ng/mL (3240 IU/mL-4000 IU/mL).

As well known, IL-2 is a glycoprotein with a molecular weight of 14-17 kDa, which is produced when T cells recognize and are activated by an antigen. Once secreted outside the T cell, IL-2 interacts with the T-cells that produce Il-2 and promotes the growth of the T cell. IL-2 also acts on NK cells to promote the growth and enhance the killing capacity of NK cells, and acts on B cells to promote their growth. L-Glutamine is a component that acts as a nutrient for immune cell culture, and the cell culture media may be known media as the basic media for floating cell culture.

As a specific example of B unit, 2 mg of IL-2 and 100 ml of 500 mM L-glutamine solution are added to the basic culture media for floating cell culture (commercially available cell culture medium, if IL-2 or L-glutamine is already present in the basic media, the amount to be added is adjusted so as to set the final concentration) and dissolved, and the final solution is adjusted to 10 L to prepare the basic solution (B unit).

C1-1 unit is composed of the cytokine 1-1 solution, which contains IL-12, IL-18 and the basic solution. Herein, IL-12 in the cytokine 1-1 solution may be contained at a concentration of 0.5 ng/mL-5 ug/mL, preferably at a concentration of 1-3 ng/mL, more preferably at 1.5 ng/mL-2.5 ng/mL. IL-18 may be included at a concentration of 2 ng/mL-50 ng/mL, preferably at a concentration of 6 ng/mL-20 ng/mL, more preferably at a concentration of 11 ng/mL-15 ng/mL.

C1-2 unit is composed of the cytokine 1-2 solution, which contains IL-12, IL-18 and the basic solution in the same manner as the cytokine 1-1 solution, except that the content of each component is different. IL-12 in the cytokine 1-2 solution may be contained at a concentration of 5.1 ng/mL-15 ug/mL, preferably at a concentration of 6-12 ng/mL, more preferably 8 ng/mL to 10.5 ug/mL. IL-18 may be included at a concentration of 30 ng/mL-120 ng/mL, preferably at a concentration of 40 ng/mL to 80 ng/mL, more preferably at a concentration of 50 ng/mL to 70 ng/mL.

As well-known, IL-12 is produced in dendritic cells (DC), macrophages, and B cells. IL-12 induces the production of IFN-γ and TNF-α in NK cells and T lymphocytes, and reduces the production of IL-4, which inhibits IFN-γ. It also increases the cytotoxicity of NK cells and CD8+ cytotoxic T lymphocytes. IL-12 is closely related to the IL-2 signaling pathway in NK cells. In addition, IL-2 induces the expression of IL-12 receptor β1 and IL-12 receptor β2 in NK cells to express and activate IL-12 signaling-related proteins. This mechanism was well demonstrated by the IFN-γ production ability and the target cell killing ability of NK cells. IL-12 receptor β2 appears to play an important role in the function of IL-12, which has been reported to inhibit the development of Th2 and concurrently promote the development of Th1. IL-12 signaling in T cells and NK cells is involved in the JAK-STAT signaling pathway and the activity of IL-12 receptor β2 plays an important role in inducing the phosphorylation and thus activation of the transcription factor STAT4.

IL-18 is also produced by macrophages and is a proinflammatory cytokine gene. IL-18 binds to the IL-18 receptor and, together with IL-12, causes an immune response to cells infected with bacteria or viruses and increases the production of IFN-γ in NK cells and T cells.

As a specific example of C1-1 unit, 10 ug of IL-12 and 50 ug of IL-18 are dissolved in water to make 10 ml and to prepare the cytokine solution (IL-12 at a concentration of 1 ug/ml, IL-18 at a concentration of 5 ug/ml). Then, 1 mL of the cytokine solution is dissolved in 500 mL of the basic solution to prepare the cytokine 1-1 solution (C1-1 unit), which contains IL-12 at a concentration of 2 ng/mL and IL-18 at a concentration of 10 ng/mL.

C2 unit is composed of the cytokine 2 solution, which has a configuration comprising IL-15 and the basic solution. The amount of IL-15 in the cytokine 2 solution may be 10-50 ng/mL, preferably 15 ng/mL-25 ng/mL.

As well known, IL-15 is a multiple expressing cytokine that belongs to the four helix bundle cytokine family. IL-15 is known to play an important role in proliferation, survival and differentiation by acting on many types of cells. In addition, IL-15 is expressed in a wider variety of cells, and thus can more broadly and more variously regulate, compared with IL-2. In particular, IL-15 affects each phase of the immune response and acts on many types of cells involved in each stage of the immune response. Results from a series of studies argue that IL-15 is directly involved in the formation of memory T cells.

As a specific example of C2 unit, the cytokine 2 solution (C2 unit) can be prepared by dissolving IL-15 in the basic solution at a concentration of 20 ng/mL.

A1 unit is composed of the antibody 1 solution, which contains the anti-CD16, the anti-CD56 and the basic solution. At this time, the content of the anti-CD16 and the anti-CD56 in the antibody 1 solution constituting A1 unit may be each 0.1-2.0 ug/mL, preferably 0.5-0.7 ug/mL.

As well known, CD16 and CD56 are the surface proteins of NK cells. When lymphocytes are cultured in the presence of the anti-CD16 antibody or an antigen-antibody complex, the CD16 antigen is added to NK cells to induce signal transduction. By the stimulation of these, NK cells express transferrin receptors, such as the a chain of the IL-2 receptor, or produce tumor necrosis factor (TNF) or IFN-γ. The anti-CD56 antibody also performs the function similar to that of the anti-CD 16 antibody.

As a specific example of A1 unit, 6 µl of the anti-CD16 and the anti-CD56 solutions (1 mg/ml) are respectively dissolved in 10 ml of the basic solution to prepare the antibody 1 solution (A1 unit).

A2 unit is composed of the antibody 2 solution, which can contain the antibody 1 solution: the basic solution in a volume ratio of 1:f, and thus, as a specific example of A2 unit, 6.5 ml of the antibody 1 solution can be mixed with 30 ml of the basic solution to prepare the antibody 2 solution.

Since A1 unit and A2 unit prepared in the state in which the antibody is already added to the basic solution can be used for 1-2 months when stored in the refrigerator, to extend the period of use, the anti-CD16, the anti-CD56 and the basic solution constituting A1 unit and A2 unit may be separately packaged and mixed in the step of adding to the media to form the antibody 1 solution and the antibody 2 solution, respectively.

D unit is composed of the antibody-cytokine mixture solution, which may contain the anti-CD3 and the cytokine 1 solution. In this case, the content of the anti-CD3 may be 1 ug/mL-12 ug/mL, preferably 3 ug/mL-8 ug/mL, and more preferably 4 ug/mL-5 ug/mL.

As well known, T cells are activated when a signal through the TCR is received, but the TCR α or β chain cannot carry the activation signal by itself and delivers the signal through the CD3 chain around the TCR. In other words, when the TCR recognizes the CD3 antigen, the CD3 antigen acts to transmit the signal into the cell. For example, when the TRC binds to the MHC+ antigen, the CD3 antigen gives a signal into the cell and the TCR forms a complex with the CD3 antigen (γ, δ, ε, ζ, ζ or η).

As a specific example of D unit, an antibody-cytokine mixture solution (D unit) can be prepared by dissolving 5 uL of the anti-CD3 solution (1 mg/mL) in 1 mL of the cytokine 1 solution.

In case of D unit, if the antibody is already added to the cytokine 1 solution, it can be used for 1-2 months when stored in the refrigerator. Therefore, to extend the period of use, the anti-CD3 and the cytokine 1 solution constituting D unit may be separately packaged and mixed in the step of adding to the media to form the antibody-cytokine mixture solution. In this case, the period of use may be extended for more than six months.

Next, the method of culturing immune cells by using a kit for adding to the media for culturing immune cells in the absence of serum of the present invention includes the Step 1 of adding at least one of the cytokine 1-1 solution of C1-1 unit and the cytokine 2 solution of C2 unit to the isolated lymphocytes, followed by addition of the autologous plasma; the Step 2 of adding the antibody 1 solution of A1 unit to the media to which the Step 1 is performed; the Step 3 of adding the antibody-cytokine mixture solution of D-unit to the media to which the Step 2 is performed; the Step 4 of adding the cytokine 1-1 solution of C1-1 unit to the media to which the Step 3 is performed, followed by addition of the autologous plasma; the Step 5 of adding the antibody 2 solution of A2 unit to the media to which the Step 4 is performed, followed by addition of the autologous plasma or FBS; the Step 6 of adding the cytokine 2 solution of C2 unit to the media to which the Step 5 is performed, followed by addition of the autologous plasma or FBS; and the Step 7 of adding the antibody 2 solution of A2 unit and the autologous plasma or FBS to the media to which the Step 6 is performed.

The method of culturing immune cells of the present invention described above can be carried out by the following two methods, such that the immune cell cultured media finally obtained may or may not contain serum depending on the application.

In the case where serum is contained in the immune cell cultured media, the method may further include the Step 8 of adding the basic solution of B unit and the autologous plasma or FBS to the media to which the Step 7 is performed, and culturing; and the Step 9 of adding the autologous plasma or FBS to the media to which the Step 8 is performed, placing into a large-capacity cell culture container containing cell culture media, and culturing.

In addition, if the immune cell cultured media does not contain serum, the method may further include the Step 10 of separating the media to which the Step 7 or the Step 9 is performed to cells and serum-containing culture media; the Step 11 of washing the cells separated in the Step 10; the Step 12 of placing the washed cells in the Step 11 into a new culture container, adding the basic solution of B unit, and culturing; and the Step 13 of placing the media to which the Step 12 is performed into a large-capacity cell culture container containing cell culture media, adding the cytokine 1-2 solution of C1-2 unit, and culturing. If necessary, the method may further include the Step 14 of separating the media to which the Step 13 is performed to cells and primary serum-free culture media; and the Step 15 of adding the basic solution of B unit to the cells separated in the Step 14, placing the cells into a large-capacity cell culture container containing cell culture media, adding the cytokine 1-2 solution of C1-2 unit, and culturing. If necessary, the Steps 14 and 15 may be repeated one or more times to obtain the secondary serum-free cultured media, tertiary serum-free cultured media, and the like. The serum-free cultured media may be obtained 3-4 liters or more in a batch by further repeating one or more times depending on the status of the cells cultured.

In this case, the lymphocytes isolated in the first step may be separated from the blood left for one day or longer after blood sampling, or may be weak immune cells, such as immune cells of the elderly or severe cancer patients. The content of the autologous plasma or FBS is less than 10% by volume of the total media.

After the Step 1 is performed and until the Step 2 is carried out, an interval of 24 hours or more can sufficiently activate the weakened immune cells. After the Step 2 is performed and until the Step 3 is carried out, the Step 3 should be performed after allowing the lymphocytes to be sufficiently stimulated by the anti-CD 16 and the anti-CD56 in the presence of the antibody 1 solution, that is, in the presence of IL-2, such that NK cells are proliferated without any particular problems although the anti-CD3 antibody included in D solution is added to the media without immobilization and stimulation is done until the anti-CD3 antibody is degraded in the culture media.

The autologous plasma added in the incubation step of the present invention contains at least three times more heparin than the amount of heparin contained in the autologous plasma used in the known culture method. In other words, even if heparin is used at a concentration of 158 usp unit per 10 mL of the blood during blood collection, the blood does not clot. However, when it is added to the cell culture at 158 usp unit per approximately 5 ml of plasma, the cells are not cultured well due to the coagulation. But, according to the present invention, it was confirmed that when the heparin was used at least three times as much as the usual amount, the cells could be cultured well without the coagulation and further, there are no problems in culturing when heparin is used in 3-4 times more amounts than what is generally known. In addition, in the plasma inactivation method that is generally used, when the proteins, such as complements, are removed by heating at 56°C for 30 minutes, the effective components necessary for cell culture are removed as well. But, when about 3 times or more of heparin is used as in the present invention and the plasma inactivation method is not used, small amounts of plasma can be used because there is no loss of the effective components necessary for cell culture. For example, 10% by volume or more of the culture media was used in the past, but it was confirmed that the culture is well performed even when only 3% by volume to 7% by volume of the media, as less than 10% by volume, was used in the present invention. Therefore, the autologous plasma used in the culture method of the present invention may contain 40 usp unit or more, preferably 50-60 usp unit or more of heparin per 1 ml of plasma.

When the immune cells are cultured in this manner, the proportion of NK cells can be increased, such that 30-80% of the immune cells cultured by the cell culture steps are NK cells. When the proportion of NK cells is increased, large amounts of cytokine, such as interleukin-8, various growth factors, interferon γ and the like can be included in the mixture cultured media. For example, the concentration of interleukin-8 may be 100-20000 pg/mL, and the concentration of interferon γ may be 400-400000 pg/mL. When the serum-free culture media contain such large amounts of interleukin-8 or interferon γ, the serum-free immune cell cultured media obtained by separating only the immune cells from the serum-free culture media can act as an active ingredient, which is highly effective for wound healing, including injuries and burn, the treatment of skin diseases, including dermatitis due to allergy, and skin care.

The cosmetic composition of the present invention is effective for at least one of skin whitening, pigmentation removal and wrinkle improvement by the action of interleukin-8 and interferon γ by including serum-free immune cell cultured media and cosmetic ingredients. Particularly, the serum-free immune cell cultured media can be used in an amount of 0.1% by weight to 50% by weight based on the total weight of the cosmetic composition, and the IL-8 may be contained in the amount of 1 pg/ml-1200 pg/ml and interferon γ may be contained in the amount of 5 pg/ml to 15000 pg/ml. As it is clear from the experimental examples described below, the inflammation improving effect is excellent in allergic rhinitis, allergic dermatitis, atopic dermatitis, acne, insect-bitten inflammation, and the like, and especially, it was effective in rapidly eliminating itching. It was found that the effect of the skin regeneration effect and the removal of pigmentation by the treatment of burns, injuries and the like was very good and it was also effective in skin whitening.

The cosmetic composition of the present invention may be prepared by using methods known in the art for making a cosmetic composition, including an emulsion, a liposome, a salt or the like, to be produced as a body lotion, a skin lotion, an essence, a cream, and the like, as well-known in the art, except that the immune cell cultured media obtained by the immune cell culture method of the present invention is included at a certain weight. Thus, a detailed description thereof will be omitted.

The pharmaceutical composition of the present invention is effective for the treatment of wounds, including burn and injuries or skin diseases by the action of interleukin-8 and interferon γ through including the serum-free immune cell cultured media as an effective ingredient. The pharmaceutical composition of the present invention can be administered in various forms of parenteral administration. When formulating the composition, it is usually formulated using diluents or excipients and the like, such as fillers, extenders, binders, wetting agents, disintegrants, surfactants, and the like that are known to be pharmaceutically acceptable.

The pharmaceutical preparations for parenteral administration include sterilized aqueous solutions, non-aqueous solvents, suspensions, emulsions and freeze-drying agents. For examples, as the skin-applicable agents, suspensions, solutions, foams, emulsions (e.g., water/oil (W/O) and oil/water (O/W) emulsions), gel preparations, and the like can be used.

In particular, the serum-free immune cell cultured media may be used in an amount of 10 wt% to 80 wt% based on the total weight of the pharmaceutical composition, in which IL-8 may be included at the amount of approximately 100 pg/mL-8000 pg/mL and interferon γ may be included at the amount of approximately 300 pg/mL-50000 pg/mL.

### EXAMPLE 1

A kit for adding to the serum-free immune cell culturing media was prepared as follows.

### 1. B unit preparation step

2.2 mg of IL-2 and 100 ml of 500 mM L-glutamine solution were added to the basic culture media for floating cell culture (if IL-2 or L-glutamine is already present in the basic media, the amount to be added is adjusted so as to set the final concentration) and dissolved, and the final solution was adjusted to 10 L to prepare the basic solution (B solution). Using 3.8 L of this solution, B unit was prepared by placing each 36 mL of the solution in a container, closing with a stopper, and labeling with a previously prepared "B solution" label, and stored as refrigerated. The remaining 6.2L was used to prepare the other units.

### 2. C1-1 unit preparation step

20 ug of IL-12 and 125 ug of IL-18 were dissolved in distilled water to make 10 mL to prepare the cytokine solution (C solution). 1ml of C solution was dissolved in 1000 mL of the basic solution (B solution) to prepare the cytokine 1-1 solution (C1-1 solution). C1-1 unit was prepared by placing each 4.5 mL of the solution in a container, closing with a stopper, and labeling with a previously prepared "C1-1 solution" label, and stored as refrigerated. The remaining 110 mL was used to prepare D unit.

### 3. C1-2 unit preparation step

5ml of C solution was dissolved in 1000 mL of the basic solution to prepare the cytokine 1-2 solution (C1-2 solution). C1-2 unit was prepared by placing each 50 mL of the solution in a container, closing with a stopper, and labeling with a previously prepared "C1-2 solution" label, and stored as refrigerated.

### 3. C2 unit preparation step

20 ug of IL-15 was added to B solution to make 1000 ml to prepare the cytokine 2 solution (C2 solution). C2 unit was prepared by placing each 9 mL of the C2 solution in a container, closing with a stopper, and labeling with a previously prepared "C2 solution" label, and stored as refrigerated.

### 4. A1 unit preparation step

0.5 mg of each of the anti-CD 16 and the anti-CD56 antibodies were dissolved in 300 mL of B solution to prepare the antibody 1 solution (A1 solution). A1 unit was prepared by placing each 1mL of 100 mL of the antibody 1 solution in a container and labeling with a previously prepared "A1 solution" label, and stored as refrigerated. The remaining 200 mL was used to prepare A2 unit.

### 5. A2 unit preparation step

200 mL of A1 solution and 3400 mL of B solution were mixed to prepare the antibody 2 solution (A2 solution). A2 unit was prepared by placing 9 mL and 27 mL of each in a container and labeling with a previously prepared "A2 solution" label, and stored as refrigerated. At this time, since A1 solution and A2 solution have a short validity period within one month, they can be prepared and used as follows.

6 uL of each of the anti-CD16 (1 mg/mL) and the anti-CD56 (1 mg/mL) antibody solutions and 3 mL of B solution are separately packaged and can be mixed and dissolved to prepare A1 and A2 solutions and use when culturing cells.

That is, first, A1 unit is prepared by placing 1 mL of A1 solution prepared by dissolving 6 uL of each of the anti-CD16 and the anti-CD56 antibodies separately packaged in 3 mL of B solution in a container and labeling with a previously prepared "A1 solution" label, and stored as refrigerated. A2 unit is prepared by mixing approximately 2 mL of the remaining solution and 34 mL of B solution to make A2 solution, separately placing 9 mL and 27 mL of the prepared A2 solution, and labeling with an "A2 solution" label, and stored as refrigerated to be used. In this case, their validity periods can be extended to more than six months.

### 6. D unit preparation step

D unit was prepared by adding 500 ug of the anti-CD3 antibody to C1-1 solution and making 105 mL to prepare the antibody-cytokine mixture solution (D solution), placing 1 mL each in a container and labeling with a previously prepared "D solution" label, and stored as refrigerated. At this time, since D solution has a short validity period within one month, it can be prepared and used as follows.

5 uL of the anti-CD3 antibody solution (1 mg / mL) and 1 mL of C1-1 solution are separately packaged in individual containers to prepare D unit, and D solution can be prepared by mixing them at the time of use. That is, before use, D unit is prepared by adding and dissolving 5 µL of the anti-CD3 antibody in 1 mL of C1-1 solution and labeling with "D1 solution, day 1" label, and stored as refrigerated. In this case, their validity periods can be extended to more than six months.

### 7. Preparation a kit for adding to the serum-free immune cell culture media

A kit for adding to the serum-free immune cell culture media can be prepared by placing B unit, C1-1 unit, C1-2 unit, C2 unit, A1 unit, A2 unit and D unit prepared. If necessary, they may be arranged in order of C1-1 unit, C2 unit, A1 unit, D unit, C1-1 unit, A2 unit, C2 unit, A2 unit, B unit and C1-2 unit according to the order of use.

### EXAMPLE 2

After lymphocytes and the autologous plasma were prepared from the blood of the patient, immune cells were cultured by using the kit for adding to the serum-free immune cell culture media as obtained in Example 1 as follows.
1. Lymphocyte extraction and autologous plasma preparation step
   Based on the characteristics that blood mononuclear cells, such as human lymphocytes or mononuclear cells, have a weight lower than 1.077, the blood of the patient to be treated was overlaid on a Ficoll-Paque Plus solution having a weight of 1.077 and centrifuged at a constant centrifugal force. Depending on the difference of the weight, the red blood cells and granulocyte layers with their weights more than 1.077 were separated down, and the mononuclear cell layer, the platelets and the like having a weight of 1.077 or less were separated up to obtain the mononuclear cell layer containing lymphocytes. From the separated mononuclear cell layer, only lymphocytes were extracted.
   More specifically, after 30 mL of the peripheral blood from patient A left for one day after blood sampling was placed in a 50 mL conical tube and centrifuged, the upper layer of the autologous plasma was placed in a heparin tube, treated and placed in a new 50 mL conical tube. After centrifugation, plasma components of the upper layer were prepared as the autologous plasma.
   Then, PBS was added to the plasma tube from which the plasma was removed to adjust the volume to 30 ml and mixed well. The mixture was transferred to a tube containing Ficoll-Paque Plus and centrifuged at 800×g for 15 minutes. The buffy coat layer containing the second layer of lymphocytes was separated, collected in a 50 ml conical tube, adjusted to a volume of 50 ml with PBS, and mixed. Thereafter, the supernatant was discarded by centrifugating 2-3 times and lymphocytes were isolated. The number of lymphocytes obtained at this time was 1.0 X 10⁷ cells .
2. Day 0: The first step of adding the autologous plasma after adding at least one of the cytokine 1-1 solution of C1-1 unit and the cytokine 2 solution of C2 unit to isolated lymphocytes was performed as follows.
   The isolated lymphocytes were treated with the C1-1 solution 1 vial (4.5 mL) and serum 0.5 mL, and placed in T25 flask and cultured in a CO₂ incubator (incubator).
3. Day 1: The second step of adding the antibody 1 solution of A1 unit to the media to which the first step was performed was performed as follows.
   The A1 solution 1 vial (1 mL) was added to the T25 flask under incubation and gently shaken, followed by continued incubation in an incubator.
4. Day 2: The third step of adding the antibody-cytokine mixture solution of D-unit to the media to which the second step was performed was performed as follows.
   The D solution 1 vial (1 mL) was added to the T25 flask under incubation and gently shaken, followed by continued incubation in an incubator.
5. Day 3: The fourth step of adding the autologous plasma, after adding the cytokine 1-1 solution of C1-1 unit to the media to which the third step was performed was performed as follows.
   The C1-1 solution (Day 3) 1 vial (4.5 mL) was added to the T25 flask under incubation, 0.5 ml of the autologous plasma was added, and gently shaken, followed by continued incubation in an incubator.
6. Day 4: The fifth step of adding the autologous plasma or FBS, after adding the antibody 2 solution of A2 unit to the media to which the fourth step was performed was performed as follows.
   By carefully scraping the bottom of the incubated T25 flask with a scraper, all cells were transferred to a T75 flask. To here, the A2 solution (Day 4) 1 vial (9 mL) and 1 ml of the autologous plasma or FBS (or something similar) were added and gently shaken, followed by continued incubation in an incubator.
7. Day 5: The sixth step of adding the autologous plasma or FBS, after adding the cytokine 2 solution of C2 unit to the media to which the fifth step was performed was performed as follows.
   The C2 solution 1 vial (9 mL) and 1 mL of autologous plasma or FBS (or something similar) were added to the T75 flask under incubation and gently shaken, followed by continued incubation in an incubator.
8. Day 6: The seventh step of adding the antibody 2 solution of A2 unit and the autologous plasma or FBS to the media to which the sixth step was performed was performed as follows.
   By carefully scraping the bottom of the incubated T75 flask with a scraper, all cells were transferred to a T150 flask. To here, the A2 solution 1 vial (27 mL) and 3 ml of the autologous plasma or FBS (or something similar) were added and gently shaken, followed by continued incubation in an incubator.
9. Day 7: The eighth step of adding the basic solution of B unit and the autologous plasma or FBS to the media to which the seventh step was performed was performed as follows.
   The B solution 1 vial (36 mL) and 4 mL of autologous plasma or FBS (or something similar) were added to the T150 flask under incubation and gently shaken, followed by continued incubation in an incubator.
10. Day 8-Day 14: The ninth step of adding the autologous plasma or FBS to the media to which the eighth step was performed, placing into a large-capacity cell culture container containing cell culture media, and culturing was performed as follows.

Day 8: Cells may be harvested at this stage by scraping the bottom of the cultured T150 flask with a scraper. However, in this study, immediately after identifying cells, 10 ml of either the autologous plasma or FBS (or something similar) is added to prepare the cell suspension. The cell suspension is added to an 1L CO₂ permeable culture bag containing cell culture media, 1/3 of which is held by forceps, massaged to be mixed well with the cell culture media, and evenly spread and incubated in a 5% CO₂ incubator at 37°C.

Day 9: The culture bag is sufficiently massaged to allow the cells to grow well, and then incubated in a 5% CO₂ incubator at 37°C.

Day 10: The forceps that hold the culture bag at 1/3 are released up to 2/3 of the bag, and the culture bag is sufficiently massaged, and then continuously incubated in a 5% CO₂ incubator at 37°C.

Day 11: The forceps that hold the culture bag at 2/3 of the bag are completely released, and the culture bag is sufficiently massaged, and then continuously incubated in a 5% CO₂ incubator at 37°C.

Day 14: Cells were harvested by placing the final cultured media in a centrifuge tube and centrifuging several times. The harvested cells can be packaged in physiological saline bags and stored as refrigerated or frozen.

### EXAMPLE 3

After the first to the seventh steps as performed in Example 2 were carried out in the same manner, the following steps were further carried out.
1. Day 7: The tenth step of separating the media to which the seventh step was performed to cells and serum-containing culture media was performed as follows.
   The cultured media of the cultured T150 flask were centrifuged to separate to immune cells and serum-containing culture media.
2. Day 7: The eleventh step of washing the cells separated in the tenth step was performed as follows.
   The immune cells centrifugated were washed with PBS more than 3 times.
3. Day 7: The twelfth step of placing the washed cells in the eleventh step into a new culture container, adding the basic solution of B unit and culturing was performed as follows.
   The washed immune cells were placed in aT150 flask, 100 ml of B solution was added thereto, and then, the cells were cultured in a CO₂ incubator.
4. Day 8-Day 11: The thirteenth step of placing the media to which the twelfth step was performed into a large-capacity cell culture container containing cell culture media, adding the cytokine 1-2 solution of C1-2 unit, and culturing was performed as follows.
   Day 8: The culture media containing cells under culture in the T150 flask were transferred to an 1L culture bag containing cell culture media, 1/2 of which was held by forceps, 50 mL of the cytokine 1-2 solution of C1-2 unit was added, and the bag was incubated in a 5% CO₂ incubator at 37°C.
   Day 9: The culture bag was sufficiently massaged to allow the cells to grow well, and then continuously incubated in a 5% CO₂ incubator at 37°C.
   Day 10: The forceps that hold the culture bag at 1/2 of the bag were removed, and the culture bag was continuously incubated.
5. Day 11 to Day 14: The fourteenth step of separating the media to which the thirteenth step was performed to cells and primary serum-free cultured media; and the fifteenth step of adding the basic solution of B unit to the cells separated in the fourteenth step, placing the cells into a large-capacity cell culture container containing cell culture media, adding the cytokine 1-2 solution of C1-2 unit, and culturing were performed as follows.
   Day 11: The primary cultured media containing cells are separated by centrifugation (400xg) to immune cells and primary serum-free cultured media, and the primary serum-free cultured media are collected and refrigerated.
   Secondary cell culture: The immune cells harvested from the primary culture media are suspended into 100 ml of B solution. The culture media containing the cells are transferred to an 1L culture bag containing cell culture media, 1/2 of which is held by forceps, 50 mL of the cytokine 1-2 solution of C1-2 unit is added, and the media are incubated in a 5% CO₂ incubator at 37°C.
   Day 12: The cells are sufficiently massaged to grow well, and then continuously incubated in a 5% CO₂ incubator at 37°C.
   Day 13: The forceps that hold the culture bag at 1/2 of the bag are removed, and the culture bag is continuously incubated.
   Day 14: The cells are sufficiently massaged to grow well, and then continuously incubated in a 5% CO₂ incubator at 37°C.
6. Day 15-Day 18: Tertiary culture after secondary cell harvest and collection of serum-free immune cell cultured media
   Day 15: The secondary cultured media containing cells subjected to the fifteenth step are separated by centrifugation (400xg) to immune cells and cultured media. The cultured media separated are stored as refrigerated as the serum-free immune cell cultured media.
   Tertiary cell culture: The immune cells harvested from the secondary culture media are suspended into 100 ml of B solution. The culture media containing the cells are transferred to an 1L culture bag containing cell culture media, 1/2 of which is held by forceps, 50 mL of the cytokine 1-2 solution of C1-2 unit is added, and the media are incubated in a 5% CO₂ incubator at 37°C.
   Day 16: The cells are sufficiently massaged to grow well, and then continuously incubated in a 5% CO₂ incubator at 37°C.
   Day 17: The forceps that hold the 1L CO₂ permeable bag at 1/2 of the bag are removed, and the culture bag is continuously incubated.
   Day 18: The cells are sufficiently massaged to grow well, and then continuously incubated in a 5% CO₂ incubator at 37°C.
7. By further repeating the steps described in 6 one or more times depending on the condition of the cells cultured, a total of 4 liters or more of serum-free cultured medium in a batch can be obtained.

### EXAMPLE 4

After the first to the eighth steps as performed in Example 2 were performed in the same manner, the following steps were further performed.
1. Day 8-Day 10: The ninth step (cell harvesting step after mass culture) of adding the autologous plasma or FBS to the media to which the eighth step was performed, placing into a large-capacity cell culture container containing cell culture media, and culturing was performed as follows.
   Day 8: All the remaining autologous plasma or FBS (or something similar) is added to the culture media. The cells containing the cells under culture are transferred to an 1L CO₂ permeable culture bag containing cell culture media, 1/2 of which is held by forceps, and the cells are incubated in a 5% CO₂ incubator at 37°C.
   Day 9: The culture bag is sufficiently massaged to allow the cells to grow well, and then continuously incubated in a 5% CO₂ incubator at 37°C.
   Day 10: The forceps that hold the 1L CO₂ permeable bag at 1/2 of the bag are removed, the culture bag is sufficiently massaged, and the culture bag is continuously incubated.
2. From Day 11, the tenth step to the fifteenth step as performed in Example 3 were performed in the same manner to obtain serum-containing cultured media, and primary serum-free cultured media to fourth serum-free cultured media.

### Comparative Example

The same method as described in Example 2 was carried out except that the first step was not carried out in Example 2 to obtain the comparative cultured media.

### Experimental Example 1

The total cell number and the proportion of NK cells in the cultured media as obtained in Example 2 and the comparative cultured media as obtained in Comparative Example were analyzed and the results are shown in Table 1.

**[Table 1]**

| Comparison | Initial cell number | Total cell number after 2 weeks of culture | Proportion of NK cells after 2 weeks of culture |
|---|---|---|---|
| Comparative Example | 1.0 x 10⁷ | 195 x 10⁷ | 40.2% |
| Example 2 | 1.0 x 10⁷ | 250 x 10⁷ | 60.5% |

Table 1 shows that for weakened immune cells, such as lymphocytes isolated from the blood left for one day after blood sampling from patients, the number of cells proliferated as well as the proportion of NK cells are increased if the lymphocytes are activated and recovered by cytokines and then stimulated with the anti-CD 16 and the anti-CD56.

### Experimental Example 2

The total number of cells cultured, the contents of interleukin-8 and interferon γ, and the proportion of NK cells in the serum-containing cultured media, the primary serum-free cultured media, the secondary serum-free cultured media, the tertiary serum-free cultured media, and the fourth serum-free cultured media as obtained in Example 3 were analyzed and the results are shown in Table 2.

Table 2 shows that even when the cells are cultured up to the fourth time, the cultured media having suitable performance in terms of the total cell number, the contents of interleukin-8 and interferon γ, and the proportion of NK cells can be obtained.

**[Table 2]**

| | Serum-containing cultured media | Primary serum-free cultured media | Secondary serum-free cultured media | Tertiary serum-free cultured media | Fourth serum-free cultured media |
|---|---|---|---|---|---|
| Culture days (days) | day7 | day11 | day15 | day19 | day23 |
| Cultured media yield (L) | 0.06 | 1.1 | 1.1 | 1.1 | 1.1 |
| Cell number (x 10⁷) | 11.5 | 120 | 150 | 140 | 125 |
| IL-8 (ng/mL) | 36.1 | 7.8 | 8.4 | 6.2 | 5.1 |
| INF-γ (ng/mL) | 1250 | 121.1 | 113.2 | 57.95 | 32.1 |
| NK cells (%) (CD16+, CD56+) | 21.2 | 34.6 | 46.72 | 56.17 | 55.2 |
| Serum (presence, absence) | Presence | Absence | Absence | Absence | Absence |

### Experimental Example 3.

The total number of cells cultured, the contents of interleukin-8 and interferon γ, and the proportion of NK cells in the serum-containing cultured media, the primary serum-free cultured media, the secondary serum-free cultured media, the tertiary serum-free cultured media, and the fourth serum-free cultured media as obtained in Example 4 were analyzed and the results are shown in Table 3.

As shown in Table 2 above and Table 3 below, when the fourth serum-free cultured media is obtained after 7 more days of culture as compared with Example 3, there were no differences in the total cell number or the proportion of NK cells, and the contents of interleukin-8 and interferon γ decrease to about the half level, perhaps since the activity of immune cells is reduced. However, according to the present invention, compared to the cultured media obtained by the conventional method, the serum-free immune cell cultured media with suitable amounts of cytokines in the absence of serum can be obtained.

**[Table 3]**

| | Serum-containing cultured media | Primary serum-free cultured media | Secondary serum-free cultured media | Tertiary serum-free cultured media | Fourth serum-free cultured media |
|---|---|---|---|---|---|
| Culture days (days) | day11 | day15 | day19 | day23 | day27 |
| Cultured media yield (L) | 1.1 | 1.1 | 1.1 | 1.1 | 1.1 |
| Cell number (x 10⁷) | 210 | 410 | 390 | 350 | 245 |
| IL-8 (ng/mL) | 3.8 | 4.4 | 4.2 | 3.8 | 2.5 |
| INF-γ (ng/mL) | 49.7 | 126.3 | 55.6 | 30.6 | 15.2 |
| NK cells (%) (CD16+, CD56+) | 34.6 | 46.72 | 56.1 | 55.7 | 56.2 |
| Serum (presence, absence) | Presence | Absence | Absence | Absence | Absence |

From the above experimental results, it was confirmed that the contents of various cytokines, which are secondary metabolites of immune cells, increase sharply at about 2 days after the media is replaced and reach an approximate peak at about 3 days. Therefore, in the present invention, the immune cell cultured media rich in the contents of cytokines can be obtained by changing the culture media at intervals of 3-4 days from the secondary cultured media. Since the immune cell cultured media of the present invention contain large amounts of cytokines and thus are very effective in skin whitening, removal of pigmentation and skin regeneration effect, they can be a competitive resource as a therapeutic agent or as a cosmetic resource.

### EXAMPLE 5

A cosmetic composition having the composition as shown in Table 4 below containing 5% by weight of the serum-free immune cell cultured media as obtained in Example 3 was prepared.

**[Table 4]**

| Ingredient | | Content (unit:% by weight) |
|---|---|---|
| Immune cell cultured media liposomes (% as cultured media) | | 5.0 |
| Cosmetic ingredient | Water | Suitable amount |
| | Dimethicone | 1,000 |
| | Cyclopentasiloxane | 3.000 |
| | Caprylic/Capric Triglyceride | 3.000 |
| | Squalane | 5.000 |
| | Butylene Glycol | 5.000 |
| | Glycereth-26 | 1,000 |
| | Dimethicone/Vinyl Dimethicone Crosspolymer | 1,000 |
| | Cetyl PEG/PPG-10/1 Dimethicone | 1,000 |
| | Mineral Oil | 2.000 |
| | Cetyl Ethylhexanoate | 3.000 |
| | Glycerin | 5.000 |
| | Beta-Glucan | 2.000 |
| | Macadamia Integrifolia Seed Oil | 0.500 |
| | Prunus Armeniaca (Apricot) Kernel Oil | 0.500 |
| | Lecithin | 0.800 |
| | Hydrogenated Lecithin | 3.000 |
| | Oligopeptide-1 | 1,000 |
| | Acetyl Hexapeptide-1 | 1,000 |
| | Stearalkonium Chloride | 0.500 |
| | Dipeptide Diaminobutyroyl Benzylamide | 0.500 |
| | Diacetate | |
| | Phenoxyethanol | 0.500 |
| | Chlorphenesin | 0.200 |
| | Sodium Hyaluronate | 0.100 |
| | Tocopheryl Acetate | 0.100 |
| | Disodium EDTA | 0.050 |
| Perfume, Preservative | | Suitable amount |
| Total | | 100 |

### Experimental Example 4

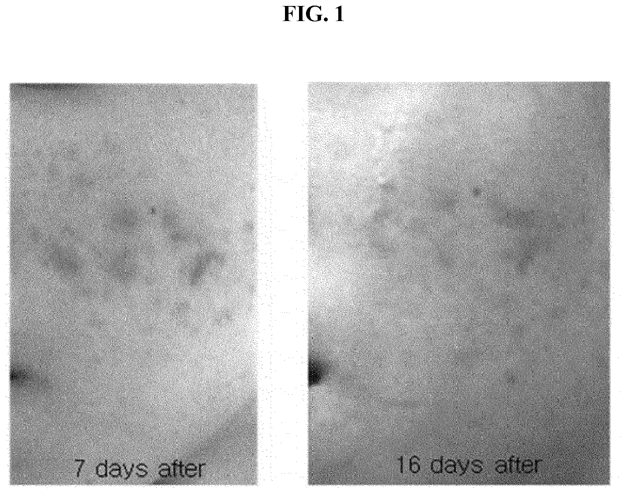

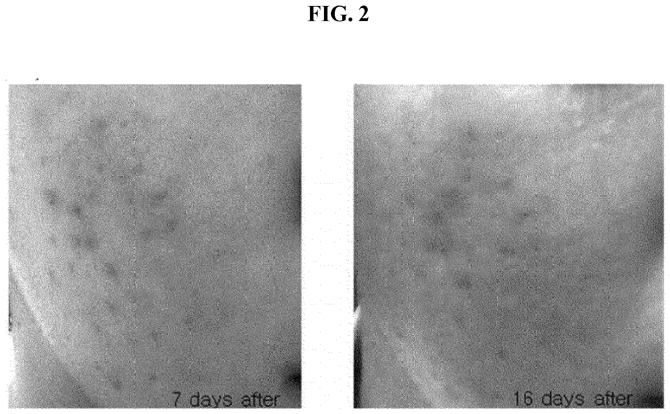

To identify the effect of improving inflammation and improving pigmentation of the cosmetic composition as obtained in Example 5, the cosmetic composition was applied twice in the morning and in the evening to the acne areas of two persons after cleansing, and photographs displaying the progress at the day 7 and at the day 16 are shown as FIG. 1 and FIG. 2, respectively.

From FIG. 1 and FIG. 2, it was found that acne was very severe at first, but acne was greatly improved over time and many deposited pigments were also removed.

### Experimental Example 5

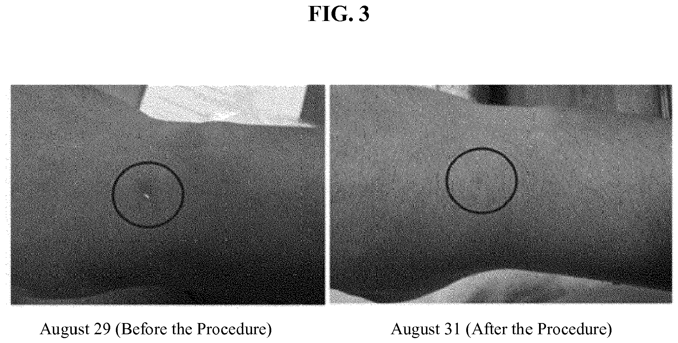

To identify the effect of improving inflammation and improving pigmentation of the cosmetic composition as obtained in Example 5, the cosmetic composition was applied twice in the morning and in the evening to the red spot area bitten by an insect alive, and photographs displaying the progress are shown as FIG. 3.

As shown in FIG. 3, it was identified that the inflammation was quickly removed, the itching was eliminated, and the red spot disappeared within two days.

### Experimental Example 6

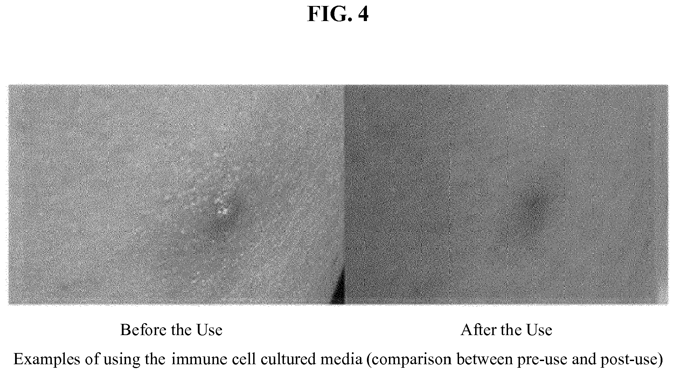

To identify the effect of improving inflammation and improving pigmentation of the cosmetic composition as obtained in Example 5, the cosmetic composition was applied twice in the morning and in the evening to the area with allergy, and photographs displaying the progress are shown as FIG. 4.

As shown in FIG. 4, it was identified that the pus was removed within one day of application, but it took about one week for the pigments to be removed, although it was not shown.

### Experimental Example 7

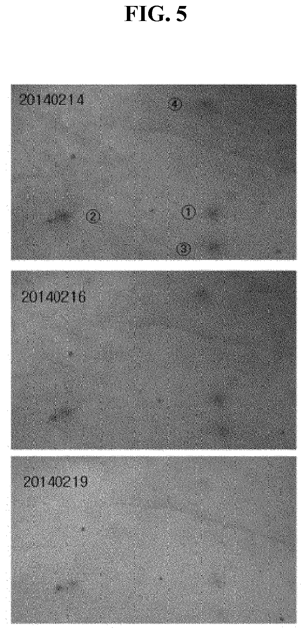

To identify the effect of improving inflammation and improving pigmentation of the cosmetic composition as obtained in Example 5, the cosmetic composition was applied twice in the morning and in the evening to the wide area where various allergies occurred with time differences, and photographs displaying the progress are shown as FIG. 5.

As shown in FIG. 5, it took about one week until inflammation and pigmentation disappeared.

### EXAMPLE 6

A pharmaceutical composition containing 20% by weight of the serum-free immune cell cultured media as obtained in Example 3 was prepared. The composition was prepared in the same manner as Table 4 of Example 3 except that 20% by weight of the serum-free immune cell cultured media was used.

### Experimental Example 8

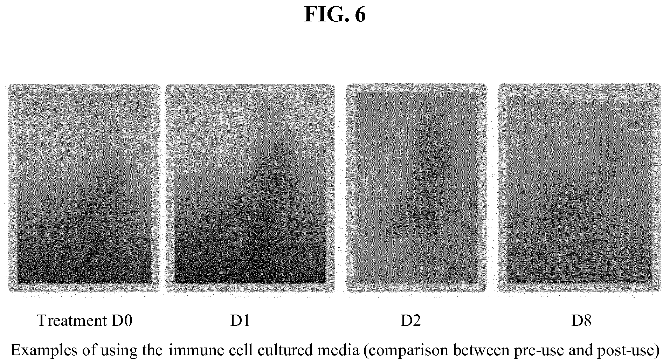

To identify the skin regeneration effect of the pharmaceutical composition as obtained in Example 6, the pharmaceutical composition was applied twice in the morning and in the evening to the burn area, and photographs displaying the progress are shown as FIG. 6.

Specifically, the diagonal wound shown in FIG. 6 was the result of the arm first burned in the baker's oven at the end of June 2016, and the vertical wound was the result of the second burn on August 31st. After the pharmaceutical composition was applied to the burn area twice in the morning and in the evening for 16 days, it was identified that the epidermis damaged was recovered and the deposited pigments were disappeared. When the pharmaceutical composition was applied right after the burn, treatment effect was much better.

While the present invention has been particularly shown and explained with reference to the preferable exemplary embodiments as described above, it is clearly understood that the invention is not limited to the exemplary embodiments described above and by those skilled in the art, various changes and modifications may be made possible within the scope of not leaving the spirit of the present invention.

## Claims

1. A kit for adding to serum-free immune cell culturing media comprising a B unit composed of a basic solution including IL-2, L-glutamine and cell culture media; a C1-1 unit composed of a cytokine 1-1 solution formed by dissolving IL-12 and IL-18 in the basic solution, such that IL-12 is included at a concentration of 0.5-5 ng/mL and IL-18 is included at a concentration of 2-50 ng/mL; a C1-2 unit composed of a cytokine 1-2 solution formed by dissolving IL-12 and IL-18 in the basic solution, such that IL-12 is included at a concentration of 5.1-15 ng/mL and IL-18 is included at a concentration of 30-120 ng/mL; a C2 unit composed of a cytokine 2 solution formed by dissolving IL-15 in the basic solution, such that IL-15 is included at a concentration of 10-50 ng/mL; an A1 unit composed of an antibody 1 solution formed by dissolving an anti-CD16 and an anti-CD56 in the basic solution, such that the anti-CD16 and the anti-CD56 are included at a concentration of 0.1-15 µg/mL each; an A2 unit composed of an antibody 2 solution comprising the antibody 1 solution:the basic solution in a volume ratio of 1:6-10; and a D unit composed of an antibody-cytokine mixture solution formed by dissolving an anti-CD3 in the cytokine 1 solution, such that the anti-CD3 is included at a concentration of 1-12 µg/mL.

2. The kit for adding to serum-free immune cell culturing media according to claim 1, wherein the kit is **characterized by** adding the A1 unit to a lymphocyte culture media prior to the D unit.

3. The kit for adding to serum-free immune cell culturing media according to claim 1, wherein the kit is **characterized by** that the anti-CD16, the anti-CD56, and the basic solution contained in the A1 unit are separately packaged and to be mixed in the step of adding to the media to form the antibody 1 solution.

4. The kit for adding to serum-free immune cell culturing media according to claim 1, wherein the kit is **characterized by** that the anti-CD 16, the anti-CD56 and the basic solution of the antibody 1 solution contained in the A2 unit and the basic solution are separately packaged and to be mixed in the step of adding to the media to form the antibody 2 solution.

5. The kit for adding to serum-free immune cell culturing media according to claim 1, wherein the kit is **characterized by** that the anti-CD3 and the cytokine 1 solution contained in the D unit are separately packaged and to be mixed in the step of adding to the media to form the antibody-cytokine mixture solution.

6. The kit for adding to serum-free immune cell culturing media according to claim 1, wherein the kit is **characterized by** that the units are arranged to be sequentially added to the lymphocyte culture media and used in order of C1-1 unit, C2 unit, A1 unit, D unit, C1-1 unit, A2 unit, C2 unit, A2 unit, B unit and C1-2 unit, order of C2 unit, C1-1 unit, A1 unit, D unit, C1-1 unit, A2 unit, C2 unit, A2 unit, B unit and C1-2 unit, order of C1-1 unit, A1 unit, D unit, C1-1 unit, A2 unit, C2 unit, A2 unit, B unit and C1-2 unit, or order of C2 unit, A1 unit, D unit, C1-1 unit, A2 unit, C2 unit, A2 unit, B unit and C1-2 unit.

7. A method of culturing an immune cell comprising a first step of adding at least one of a cytokine 1-1 solution of a C1-1 unit and a cytokine 2 solution of a C2 unit to an isolated lymphocyte, followed by addition of an autologous plasma; a second step of adding an antibody 1 solution of an A1 unit to a media to which the first step is performed; a third step of adding an antibody-cytokine mixture solution of a D-unit to the media to which the second step is performed; a fourth step of adding the cytokine 1-1 solution of the C1-1 unit to the media to which the third step is performed, followed by addition of the autologous plasma; a fifth step of adding an antibody 2 solution of an A2 unit to the media to which the fourth step is performed, followed by addition of the autologous plasma or a FBS (fetal bovine serum); a sixth step of adding a cytokine 2 solution of a C2 unit to the media to which the fifth step is performed, followed by addition of the autologous plasma or the FBS; and a seventh step of adding the antibody 2 solution of the A2 unit and the autologous plasma or the FBS to the media to which the sixth step is performed.

8. The method of culturing an immune cell according to claim 7, further comprising an eighth step of adding the basic solution of the B unit and the autologous plasma or the FBS to the media to which the seventh step is performed, and culturing; and a ninth step of adding the autologous plasma or the FBS to the media to which the eighth step is performed, placing into a large-capacity cell culture container containing a cell culture media, and culturing.

9. The method of culturing an immune cell according to claim 7 or 8, wherein the isolated lymphocyte is isolated from the blood left for one day or more after blood collection.

10. The method of culturing an immune cell of claim 7 or 8, further comprising a tenth step of separating the media to which the seventh step or the ninth step is performed to cells and serum-containing cultured media; an eleventh step of washing the cells separated in the tenth step; a twelfth step of placing the washed cells in the eleventh step into a new culture container, adding the basic solution of the B unit, and culturing; and a thirteenth step of placing the media to which the twelfth step is performed into a large-capacity cell culture container containing cell culture media, adding the cytokine 1-2 solution of C1-2 unit, and culturing.

11. The method of culturing an immune cell of claim 10, further comprising a fourteenth step of separating the media to which the thirteenth step is performed to cells and serum-free primary cultured media; and a fifteenth step of adding the basic solution of the B unit to the cells separated in the fourteenth step, placing the cells into a large-capacity cell culture container containing cell culture media, adding the cytokine 1-2 solution of the C1-2 unit, and culturing.

12. The method of culturing an immune cell according to claim 7 or 8, wherein the autologous plasma contains more than 40 usp units of heparin per 1 ml of the plasma.

13. The method of culturing an immune cell of claim 7 or 8, wherein a content of the autologous plasma or the FBS added is less than 10% by volume of the total media.

14. An immune cell cultured media cultured by the method of culturing an immune cell according to claim 7 or 8.

15. A serum-free immune cell cultured media obtained from an immune cell cultured media cultured using the kit for adding to the serum-free immune cell culturing media according to claim 1 or 6, or by the method of culturing an immune cell according to claim 10.

16. A cosmetic composition comprising the serum-free immune cell cultured media according to claim 15 and a cosmetic base.

17. The cosmetic composition according to claim 16, wherein the cosmetic composition is **characterized by** that the serum-free immune cell cultured media function as an active ingredient for at least one of inflammation improvement, skin whitening, pigmentation removal and skin regeneration.

18. The cosmetic composition according to claim 16, wherein the cosmetic composition is **characterized by** that the serum-free immune cell cultured media is contained in an amount of 50% by weight or less based on the weight of the total composition.

19. A pharmaceutical composition for treating a wound including a burn and an injury or a skin disease comprising the serum-free immune cell cultured media according to claim 15.
